(19) Europäisches Patentamt
European Patent Office
Office européen des brevets

(11) Publication number: **0 031 176**
**B1**

(12) # EUROPEAN PATENT SPECIFICATION

(45) Date of publication of patent specification: **14.03.84**

(51) Int. Cl.³: **C 07 D 209/52, A 01 N 43/38**

(21) Application number: **80201141.1**

(22) Date of filing: **02.12.80**

(54) **Process for conversion of certain trans 3,4-methano-pyrrolidine derivatives into their corresponding cis isomers, a novel unsaturated intermediate in the process and derivatives thereof, a process for preparing the novel intermediate and derivatives thereof, pollen-suppressing compositions and a method of producing F1 hybrid seed.**

(30) Priority: **19.12.79 GB 7943797**

(43) Date of publication of application:
**01.07.81 Bulletin 81/26**

(45) Publication of the grant of the patent:
**14.03.84 Bulletin 84/11**

(84) Designated Contracting States:
**BE CH DE FR GB IT LI NL SE**

(56) References cited:
**EP - A - 0 010 787**
**EP - A - 0 012 470**

(73) Proprietor: **SHELL INTERNATIONALE RESEARCH MAATSCHAPPIJ B.V.**
*Carel van Bylandtlaan 30*
**NL-2596 HR Den Haag (NL)**

(72) Inventor: **Mason, Ronald Frank**
**"Kingswood" Westwell**
**Ashford Kent (GB)**
Inventor: **Wood, Derek Alexander**
**76 Sterling Road**
**Sittingbourne Kent (GB)**

(74) Representative: **Hunter, Keith Roger Ian et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 031 176 B1**

# Process for conversion of certain trans 3,4-methano-pyrrolidine derivatives into their corresponding cis isomers, a novel unsaturated intermediate in the process and derivatives thereof, a process for preparing the novel intermediate and derivatives thereof, pollen-suppressing compositions and a method of producing $F_1$ hybrid seed

This invention relates to a process for the isomerisation of derivatives of 3-azabicyclo-[3.1.0]hexane, and to novel compositions useful as pollen suppressants.

2-Carboxy-azabicyclo[3.1.0]hexane and certain derivatives thereof have exhibited extremely useful plant growth regulating and pollen suppressing properties. The single isomer cis (L) 2-carboxy-3-azabicyclo[3.1.0]hexane is found in the seeds of the American horse chestnut in small amounts, and much research has been directed to developing a synthetic method of producing this and the other isomers in larger amounts.

A method has now been found by which trans 2-carboxy-3-azabicyclo[3.1.0]hexane and derivatives thereof can be converted into the corresponding cis isomer.

The invention provides a process for the convertion of the trans isomer of an acid of the formula

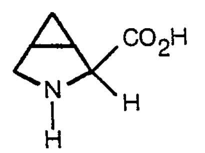

(I)

or a salt, ester, or salt of an ester thereof, into the cis isomer of such a compound, which comprises converting the trans isomer into an acid of the formula

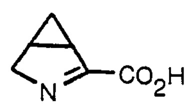

or a salt thereof, and subsequently hydrogenating said compound; and, in the event of an ester or salt thereof being required converting the resulting free acid or salt thereof into the desired ester or salt thereof.

Various possibilities of isomerism exist, of course, depending upon the nature of substitutents on the bicyclic nucleus. However, throughout this specification, a trans isomer should be understood to be a geometric isomer in which the $—CO_2—$ group in the 2-position of the 3-azabicyclohexane ring is trans to the bridging group in the 6-position, and a cis isomer is a geometric isomer in which the $—CO_2—$ group is cis to the bridging group.

It should be noted that for each geometric isomer, a pair of optical isomers exists, due to the asymmetry of the 2-carbon atom.

The starting trans compound may be either pure trans isomer or it may be present in a mixture of cis and trans isomers. Thus any mixture of cis and trans isomers may be converted into cis compounds, or into a mixture of trans and cis compounding containing an enhanced proportion of cis isomer, by the process of the invention.

If desired, the product obtained directly from the hydrogenation process, which is an acid or a salt thereof, may be converted into an ester or a salt thereof. This may be performed by any suitable method known for the preparation of esters and salts thereof.

The starting material may be a free acid I or a salt thereof, for example an alkali metal salt or a hydrohalide salt. Preferably, however, an ester is used as starting material. The ester may for example by an alkyl, alkenyl, alkynyl, aryl or aralkyl ester in which the ester moiety may be unsubstituted or substituted by one or more of the same or different substituents selected from halogen atoms, alkyl groups and alkoxy groups. Preferably the ester is an alkyl, alkenyl ester in which the ester moiety is unsubstituted. More preferably the ester is an alkyl ester having from 1 to 10, especially 1 to 4 carbon atoms in the alkyl group. For example, the ester may be a methyl, ethyl or isopropyl ester.

If an ester or salt thereof, for example a hydrohalide salt thereof, is used as starting material, it is suitably converted to the acid II or salt thereof in two stages. Firstly, it is converted into an ester of the acid II, and secondly, the ester is hydrolysed. The conversion of the starting ester to an ester of the acid II may for example be carried out directly using an oxidising agent. Manganese dioxide is a suitable reagent, and the oxidation can be performed simply by stirring the ester of the compound of formula II with manganese dioxide in the presence of a suitable solvent, for example a hydrocarbon such as benzene or light petroleum. The reaction is conveniently performed at room temperature. Alternatively, the conversion may be carried out indirectly for example by chlorinating or brominating the ester of an acid of the formula I to give an ester of an acid of the formula

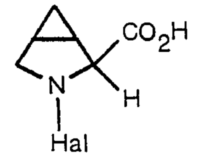

(III)

in which Hal is a chlorine or bromine atom, and subsequently dehydrohalogenating the N-halo compound. The halogenation may be carried out using any suitable halogenating agent, for example N-bromo- or, especially, N-chlorosuccinimide, or an organic or inorganic hypohalite, for example t-butyl hypochlorite or

sodium hypochlorite. Sodium hypochlorite may conveniently be used in the form of sodium hydroxide plus chlorine. The halogenation is suitably carried out by admixing the halogenating agent with the starting ester. Any suitable solvent, for example an ether, may be used. Water is a suitable solvent when using an inorganic halogenating agent. The reaction may for example by carried out at a temperature in the range of from −10°C to +30°C, the optimum temperature depending on the halogenating agent used. For example, if a hypohalite is used as halogenating agent, the reaction is preferably carried out at a temperature in the range of from −10°C to +5°C, whereas if N-chlorosuccinimide is used as halogenating agent, the reaction is most conveniently carried out at room temperature.

Suitable dehydrohalogenating agents for the dehydrohalogenation step include organic bases and inorganic bases, for example an alkali metal hydroxide or alkoxide, or a tertiary amine, for example triethylamine. If it is desired to isolate the ester of the acid of the formula II, care should be taken to ensure that the reaction conditions are such that the ester group is not attacked. For this reason, the base used should be relatively non-nucleophilic; for example, sodium ethoxide is generally preferred to sodium hydroxide. The reaction may be carried out in any suitable polar solvent, for example an ether or an alcohol, and is preferably carried out at a temperature of up to 150°C, preferably at a temperature in the range 0°C to 80°C.

The resulting azabicyclohexene ester may be isolated by any suitable method, or at least part of the reaction mixture containing the azabicyclohexene ester may be used directly in the subsequent hydrolysis step.

In the subsequent hydrolysis step, the resulting ester of the acid of the formula II is converted into the free acid II or salt thereof by hydrolysis. The hydrolysis may be carried out by any of the methods normally used for the hydrolysis of esters, for example using acidic or basic catalysts, and whether an acid or a salt is formed depends of course on the conditions. In a preferred embodiment of the process according to the invention, an ester and not a salt of an ester is used as starting material, and the hydrolysis is carried out using water only, to avoid the introduction of inorganic ions into the reaction mixture. Such a procedure may facilitate workup of the final product.

In an alternative embodiment of the process of the invention the ester of the acid of the formula III is prepared, and then dehydrohalogenated under conditions which also cause hydrolysis of the ester group. The use of a relatively nucleophilic base, for example sodium hydroxide, facilitates such a process.

If the free acid of the formula I, or a salt thereof, is used as starting material, it may be directly oxidised to the acid of the formula II or a salt thereof, or it may be converted indirectly by the process of N-chlorination or N-bromination followed by dehydrohalogenation as described above for the conversion of esters. Conditions for the direct oxidation or for the N-halogenation and subsequent dehydrohalogenation of an acid or salt are as described above for esters, except that it may be preferable to work in a reaction medium which consists of or includes water.

The hydrogenation step of the process according to the invention may for example be carried out by using a hydride transfer agent; preferably however it is carried out using gaseous hydrogen in the presence of a homogeneous or, preferably, heterogeneous hydrogenation catalyst, for example a palladium or platinum catalyst, such as palladium charcoal or, preferably, platinum charcoal. The process may be carried out at atmospheric pressure but it is preferably carried out at elevated pressure, for example up to 150 atmospheres gauge.

The hydrogenation reaction is preferably carried out at room temperature. It may however if desired be carried out at elevated temperature, for example up to 100°C. Any suitable solvent may be used. Water is especially preferred.

Depending upon conditions, the monohydrate of an acid of the formula II may exist wholly or partly in tautomeric form. In aqueous solution, it is believed that an equilibrium exists between the bicyclic structure and the open chain structure:

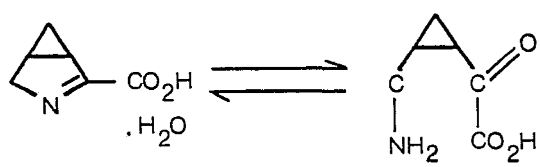

This equilibrium may also exist under certain conditions in other solvents and even in the solid state. Elemental analysis alone cannot of course distinguish between the monohydrate of the bicyclic acid, and the open chain compound, but spectroscopic methods such as NMR can be used to detect either form. Throughout this specification and claims, it should be understood that any reference to the bicyclic acid or salt thereof includes the possibility that the acid or salt may in fact be present wholly or partly as the open-chain tautometer.

Esters of the acid of the formula II are believed to exist very largely or entirely by bicyclic form.

The acid 2-carboxy-3-azabicyclo[3.1.0]hex-2-ene or a salt and/or amide, alkylamide, hydrazide or alkylhydrazide thereof may be used to sterilise the male anthers of plants, especially small-grain cereal plants, without substantially affecting female fertility. This makes it possible to produce $F_1$ hybrids of self-pollinating plants using a simple chemical treatment. The invention therefore provides a pollen suppressing composition which comprises said acid or

derivative thereof together with a suitable carrier. The invention further provides a method of sterilising the male anthers of a plant, which comprises applying to the plant said acid or derivative thereof or a pollen-suppressing composition containing such a compound. The invention also provides a method of producing an $F_1$ hybrid seed which comprises cross-pollinating a plant which has been treated by the sterilising process according to the invention with a second plant of a different strain.

Preferably the active compound or composition is applied to a small-grain cereal plant, for example wheat or barley, when the plant is at a stage in growth between late tillering and emergence of the ear. The compound or composition is suitably applied at a dosage of active compound of from 0.05 to 2 kg/ha, preferably 0.25 to 1 kg/ha.

Suitable salts of 2-carboxy-3-azabicyclo-[3.1.0]hex-2-ene include hydrohalide and alkali metal salts. In an alkyl amide or alkylhydrazide, the or each alkyl group preferably has up to 4 carbon atoms. In a preferred embodiment of the invention, there is provided the monohydrate of 2-carboxy-3-azabicylo[3.1.0]hex-2-ene.

The invention also provides a process for the preparation of 2-carboxy-3-azabicyclo[3.1.0]-hex-2-ene or a salt and/or an amide, alkylamide, hydrazide or alkylhydrazide thereof, which comprises oxidising 2-carboxy-3-azabicyclo[3.1.0]hexane or a salt and/or an ester thereof, and if the starting material is an ester, hydrolysing the resulting ester of 2-carboxy-3-azabicyclo[3.1.0]hex-2-ene, and in the event of an amide, alkylamide, hydrazide or alkylhydrazide being required, converting the resulting acid or salt into the desired compound.

The invention also provides a process for the preparation of the cis isomer of 2-carboxy-3-azabicyclo[3.1.0]hexane or a salt thereof, which comprises hydrogenating 2-carboxy-3-azabicyclo[3.1.0]hex-2-ene or a salt thereof.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating agricultural compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface active agent.

A surface-active agent may be an emulsifying agent, a dispersing agent or a wetting agent; it may be nonionic or ionic. Examples of suitable surface-active agents include the sodium or calcium salts or polyacrylic acids and lignin sulphonic acids; the condensation products of fatty acids or aliphatic amines or amines containing at least 12 carbon atoms in the molecule with ethylene oxide and/or propylene oxide; fatty acid esters of glycerol, sorbitan, sucrose, or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifyable concentrates, emulsions, suspension concentrates and aerosols. Wettable powders usually contain 25, 50 and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a

wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—25% w active ingredient and 0—10% w of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents,, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing insecticidal herbicidal or fungicidal properties.

The following Examples illustrates the invention.

Example 1

*Conversion of trans 2-ethoxycarbonyl-3-azabicyclo[3.1.0]hexane into cis 2-carboxy-3-azabicyclo[3.1.0]hexane*

(a) Oxidation of starting ester (Method (i))

A mixture of the cis and trans isomers (ratio 55:45) of 2-ethoxycarbonyl-3-azabicyclo-[3.1.0]hexane (62 g, 0.4 m) was added over 2 hours to N-chlorosuccinimide (56.0 g, 0.42 mol) in diethyl ether (30 ml) under a nitrogen atmosphere, the mixture being cooled with ice to maintain the internal temperature at 18—21°C. The mixture was then filtered, and triethylamine (60.6 g, 0.6 mol) was added to the filtrate over 30 minutes. The mixture was then heated to reflux for 6 hours, and allowed to stand overnight at room temperature. It was then filtered, and on evaporation 66.3 g of crude 2 - ethoxycarbonyl - 3 - azabicyclo-[3.1.0]hex-2-ene was obtained. The crude product was distilled to give 42.5 g of the pure product which had a boiling point of 56—58°C at a pressure of 0.3—0.4 mm Hg.

(b) Oxidation of starting ester (Method (ii))

The mixture of cis and trans isomers described in (a) above (50 g) was dissolved in n-hexane (250 ml) and manganese dioxide (200 g) was added in four portions over 2 hours, the mixture being stirred under a nitrogen atmosphere. The internal temperature rose to 35°C. Stirring was continued overnight. A further portion of manganese dioxide was then added, and stirring was continued for a further 2 days. The mixture was then filtered and the solvent was stripped on a rotary evaporator to give a rather viscous crude product. This was distilled to give 32.7 g of the desired product.

(c) Hydrolysis of intermediate ester

19.5 g (0.127 M) of 2 - ethoxycarbonyl - 3 - azabicyclo[3.1.0]hex - 2 - ene prepared as described in either (a) or (b) above, was added to the demineralised water (175 ml) and the mixture was heated at reflux under a nitrogen atmosphere for two hours. Ethanol distilled off over this period. The mixture was then allowed to stand at room temperature overnight. Water was removed on a rotary evaporator until about 75 ml of mixture remained. Isopropyl alcohol (100 ml) was added, and the precipitate which formed on cooling was filtered off and washed first with isopropyl alcohol and then with hexane. After drying in an oven overnight at 60°C, 13.2 g of a white solid, melting point 124—126°C (with decomposition) were obtained.

| *Elemental Analysis* | C | H | N |
|---|---|---|---|
| Calculated for $C_6H_9O_3N$ | 50.3 | 6.3 | 9.8 |
| Found | 50.4 | 6.3 | 9.8 |

The elemental analysis conforms to $C_6H_9O_3N$, the empirical formula of the tautomers:

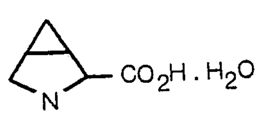 and 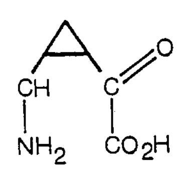

The electron impact mass spectrum of the solid, indicated that the solid was largely or entirely the bicyclic structure. The NMR spectrum in deuterated dimethylsulphoxide indicated only the bicyclic structure. The NMR spectrum in $D_2O$ solution, however, indicated the presence of both the bicyclic and the open-chain structure.

(d) Hydrogenation of product from (c)

0.2 g of Adam's Catalyst (platinum oxide) were added to demineralised water (100 ml) in a Parr Shaker. The mixture was flushed with

nitrogen and then with hydrogen at 1 atmosphere gauge pressure. The product from step (c) above (6 g) was added and the hydrogen pressure was increased to 4 atmospheres gauge. The mixture was shaken for 3.75 hours. The catalyst was filtered off, and the water was removed until a sticky residue remained. This residue was treated with isopropyl alcohol (75 ml), and the resulting crystalline precipitate was filtered off and dried overnight in an oven at 65—70°C. 4.6 g of 2 - carboxy - 3 - azabicyclo[3.1.0]hexane, melting at 245—246°C (with decomposition) were obtained. NMR showed that the product was the cis isomer, with isomeric purity of greater than 95%.

Example 2

*Demonstration of pollen-suppressing activity*

Spring wheat, variety sicco, was propagated in a glass-house in 13 cm pots containing a loam-based compost. Supplementary lighting was provided by high-pressure mercury vapor lamps to give a constant day length of 16 hours. The temperature was maintained at approximately 20°C

2 - carboxy - 3 - azabicyclo[3.1.0]hex - 2 - ene prepared by steps (a) and (c) of Example 1 was formulated as an aqueous solution containing 0.1% nonidet P40 (trade mark) as wetting agent and 1% acetone to aid solubility. This formulation was diluted with water to various concentrations and sprayed onto plants to run-off. The plants were treated at the growth stage when the second node of the plant was just detectable.

At ear emergence but before anthesis, 5 heads from each treated pot were placed in cellophane bags to prevent cross-pollination. At maturity, the bagged ears were harvested, and the seed set on both the main stems and tillers was recorded and compared with untreated controls.

The results are shown in the following Table.

TABLE

| Dosage ppm | Reduction in seed set (% relative to control) Main Stems | Tillers |
|---|---|---|
| 200 | 7 | 0 |
| 1000 | 79 | 86 |

**Claims**

1. A process for the conversion of the trans isomer of an acid of the formula:—

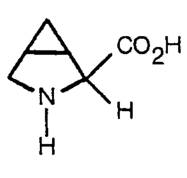

(I)

or a salt, ester, or salt of an ester thereof, into the cis isomer of such a compound whereby in the said trans and cis isomer the —$CO_2$— group is trans and cis, respectively, to the bridging group in the 6-position of the said compound, which process comprises converting the trans isomer into an acid of the formula:—

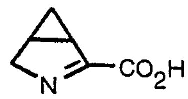

which, in the event of an aqueous solution, may at least partly be present in the open-chain tautomer form, or a salt of the said acid, and subsequently hydrogenating said compound; and, in the event of an ester or salt thereof being required, converting the resulting free acid or salt thereof into the required ester or salt thereof.

2. A process as claimed in Claim 1, characterised in that the starting material is an ester of an acid whose carbon skeleton includes the unit I.

3. A process as claimed in Claim 2, characterised in that the ester is an alkyl ester having 1 to 4 carbon atoms in the alkyl group.

4. A process as claimed in either Claim 2 or Claim 3, characterised in that the starting ester is converted into an ester of the acid of formula II either directly using an oxidising agent or by chlorination or bromination of the starting ester to produce an ester of an acid of formula

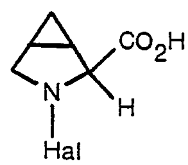

(III)

in which Hal is a chlorine or bromine atom, and subsequently dehydrohalogenating the N-halo compound; and subsequently the resulting ester is hydrolised to produce an acid of formula II or a salt thereof.

5. A process as claimed in Claim 4, characterised in that the starting ester is chlorinated using N-chloro succinimide, t-butyl hypochlorite or sodium hypochlorite.

6. A process as claimed in Claim 1, characterised in that a free acid of formula I or a salt thereof, is N-chlorinated using sodium hypochlorite, and subsequently dehydrohalogenated to produce an acid of formula II or a salt thereof.

7. A process as claimed in any one of Claims 4 to 6, characterised in that the halogenation is carried out at a temperature in the range of from −10°C to +30°C.

8. A process as claimed in any one of Claims 4 to 7, characterised in that the dehydrohalogenation step is carried out using an alkali metal hydroxide or alkoxide, or a tertiary amine.

9. A process as claimed in any one of Claim 4 to 8, characterised in that the dehydrohalogenation step is carried out at the temperature of up to 150°C.

10. A process as claimed in Claim 4, characterised in that the starting ester is directly oxidised using manganese dioxide.

11. A process as claimed in any one of Claims 1 to 10, characterised in that the hydrogenation step is carried out using gaseous hydrogen in the presence of an heterogeneous hydrogenation catalyst.

12. A process as claimed in Claim 11, characterised in that the catalyst comprises palladium or platinum.

13. A pollen suppressing composition which comprises 2 - carboxy - 3 - azabicyclo-[3.1.0]hex - 2 - ene or a salt and/or amide, alkylamide, hydrazide or alkylhydrazide thereof, together with a carrier.

14. A composition as claimed in Claim 13, which comprises at least one surface-active agent.

15. A method of sterilising the male anthers of a plant, characterised in that it comprises applying to the plant 2 - carboxy - 3 - azabicyclo[3.1.0]hex - 2 - ene or a salt and/or amide, alkylamide, hydrazide of alkylhydrazide thereof, or a composition as claimed in either Claim 13 or Claim 14.

16. A method of producing $F_1$ hybrid seed, characterised in that it comprises cross-pollinating a plant which has been treated by a process as claimed in Claim 15, which a second plant of a different strain.

17. A process for the preparation of 2 - carboxy - 3 - azabicyclo[3.1.0]hex - 2 - ene or a salt and/or an amide, alkylamide, hydrazide or alkylhydrazide thereof, which comprises oxidising 2 - carboxy - 3 - azabicyclo[3.1.0]-hexane or a salt and/or an ester thereof, and if the starting material is an ester, hydrolysing the resulting ester of 2 - carboxy - 3 - azabicyclo[3.1.0]hex - 2 - ene, and in the event of an amide, alkylamide, hydrazide or alkylhydrazide being required, converting the resulting acid or salt into the desired compound.

18. A process for the preparation of the cis isomer of 2-carboxy - 3 - azabicyclo[3.1.0]-hexane or a salt thereof, which comprises hydrogenating 2 - carboxy - 3 - azabicyclo-[3.1.0]hex - 2 - ene or a salt thereof.

19. 2 - Carboxy - 3 - azabicyclo[3.1.0]-hex - 2 - ene or a salt and/or amide, alkylamide, hydrazide or alkylhydrazide thereof.

20. The monohydrate of 2 - carboxy - 3 - azabicyclo[3.1.0]hex - 2 - ene.

**Patentansprüche**

1. Verfahren zur Umwandlung des trans-Isomeren einer Säure der Formel

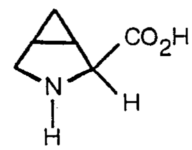

(I)

oder eines Salzes, Esters oder Salzes eines Esters davon in das cis-Isomere einer solchen Verbindung, wobei sich bei dem erwähnten trans- und cis-Isomeren die —$CO_2$—Gruppe in trans-bzw. cis-Stellung zu der Brückengruppe in 6-Stellung der Verbindung befindet, umfassend die Umwandlung des trans-Isomeren in eine Säure der Formel,

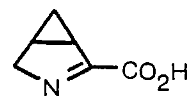

die im Falle einer wäßrigen Lösung zumindest teilweise in der tautomeren offenkettigen Form vorliegen kann, oder ein Salz dieser Säure und anschließende Hydierung dieser Verbindung und, falls eine Ester oder Salz davon erforderlich ist, Umwandlung der erhaltenen freien Säure oder des Salzes davon in den gewünschten Ester oder ein Salz davon.

2. Verfahren nach Anspruch 1, dadurch gekennzeichnet, daß das Ausgansmaterial ein Ester oder eine Säure ist, deren Kohlenstoffgerüst die Einheit I umfaßt.

3. Verfahren nach Anspruch 2, dadurch gekennzeichnet, daß der Ester ein Alkylester mit 1 bis 4 Kohlenstoffatomen in der Alkylgruppe ist.

4. Verfahren nach Anspruch 2 oder 3, dadurch gekennzeichnet, daß der Ausgangsester in einen Ester der Säure der Formel II umgewandelt wird entweder direkt mit Hilfe eines Oxidationsmittels oder durch Chlorierung oder Bromierung des Ausgansesters unter Bildung eines Esters einer Säure der Formel

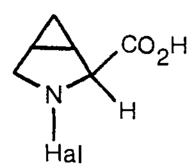

(III)

in der Hal ein Chlor- oder Bromaton bedeutet, und anschließende Dehydrohalogenierung der N-Halogenverbindung und der erhaltene Ester anschließend under Bildung einer Säure der Formel II oder eines Salzes davon hydrolysiert wird.

5. Verfahren nach Anspruch 4, dadurch gekennzeichnet, daß der Ausgangsester mit Hilfe von N-Chlor-succinimid, tert.-Butyl-hypochlorit oder Natrium-hypochlorit chloriert wird.

6. Verfahren nach Anspruch 1, dadurch gekennzeichnet, daß eine freie Säure der Formel I oder ein Salz davon mit Hilfe von Natriumhypochlorit N-chloriert und anschließend unter Bildung einer Säure der Formel II oder eines Salzes davon dehydrohalogeniert wird.

7. Verfahren nach einem der Ansprüche 4 bis 6, dadurch gekennzeichnet, daß die Halogenierung bei einer Temperatur im Bereich von —10 bis +30°C durchgeführt wird.

8. Verfahren nach einem der Ansprüche 4 bis 7, dadurch gekennzeichnet, daß die Dehydrohalogenierung mit Hilfe eines Alkalihydroxids oder-alkoxids oder eines tert. Amins durchgeführt wird.

9. Verfahren nach einem der Ansprüche 4 bis 8, dadurch gekennzeichnet, daß die Dehydrohalogenierung bei einer Temperatur von bis zu 150°C durchgeführt wird.

10. Verfahren nach Anspruch 4, dadurch gekennzeichnet, daß der Ausgangsester mit Hilfe von Mangandioxid direkt oxidiert wird.

11. Verfahren nach einem der Ansprüche 1 bis 10, dadurch gekennzeichnet, daß die Hydrierung mit Hilfe von gasförmigen Wasserstoff in Gegenwart eines heterogenen Katalysators durchgeführt wird.

12. Verfahren nach Anspruch 11, dadurch gekennzeichnet, daß der Katalysator Palladium oder Platin umfaßt.

13. Ein Pollen unterdrückendes Mittel, umfassend 2 - Carboxy - 3 - azabicyclo-[3,1,0]hex - 2 - en oder ein Salz und/oder Amid, Alkylamid, Hydrazid oder Alkylhydrazid davon zusammen mit einem Träger.

14. Mittel nach Anspruch 13, umfassend zumindest zwei Träger, von denen zumindest einer ein oberflächenaktives Mittel ist, umfaßt.

15. Verfahren zur Sterilisierung der (männlichen) Staubbeutel eine Pflanze, gekennzeichnet dadurch, daß man auf die Pflanze 2 - Carboxy - 3 - azabicyclo[3,1,0]hex - 2 - en oder ein Salz und/oder Amid, Alkylamid, Hydrazid oder Alkylhydrazid davon oder ein Mittel nach Anspruch 13 oder Anspruch 14 aufbringt.

16. Verfahren zur Erzeugung von $F_1$ Hybridsamen, dadurch gekennzeichnet, daß es das kreuzweise Bestäuben (Kreuzen) einer Pflanze umfaßt, die mit Hilfe eines Verfahrens wie in Anspruch 15 definiert, behandelt worden ist, mit einer zweiten Pflanze eines unterschiedlichen Stammes.

17. Verfahren zur Herstellung von 2 - Carboxy - 3 - azabicyclo[3,1,0]hex - 2 - en oder eines Salzes und/oder eines Amids, Alkylamids, Hydrazids oder Alkylhydrazids davon, umfasend die Oxidation von 2 - Carboxy - 3 - azabicyclo[3,1,0]hexan oder eines Salzes und/oder eines Esters davon und, wenn das Ausgangsmaterial ein Ester ist, Hydrolysieren des erhaltenen Esters von 2 - Carboxy - 3 - azabicyclo[3,1,0]hex - 2 - ene und, wenn ein Amid, Alkylamid, Hydrazid oder Alkylhydrazid erwünscht ist, Umwandlung der erhaltenen Säure oder des Salzes in die gewünschte Verbindung.

18. Verfahren zur Herstellung des cis-Isomeren von 2 - Carboxy - 3 - azabicyclo-[3,1,0]hexan oder eines Salzes davon, umfassend die Hydrierung von 2 - Carboxy - 3 - azabicyclo[3,1,0]hex - 2 - en oder eines Salzes davon.

19. 2 - Carboxy - 3 - azabicyclo[3,1,0]-hex - 2 - en oder eine Salz und/oder Amid, Alkylamid, Hydrazid oder Alkylhydrazid davon.

20. Monohydrat von 2 - Carboxy - 3 - azabicyclo[3,1,0]hex - 2 - en.

**Revendications**

1. Un procédé pour la conversion de l'isomère tans d'un acide de la formule:

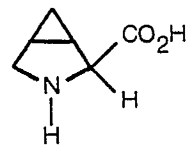

(I)

ou d'un sel, ester ou sel d'un ester de cet acide, en l'isomère cis d'un tel composé, les isomères trans et cis étant tels que le groupe $—CO_2—$ soit en relation trans et cis, respectivement, avec le groupe formant pont dans la position 6 du composé, procédé selon lequel on transforme l'isomère trans en un acide de la formule:

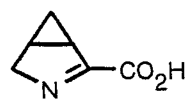

qui, dans le cas d'une solution aqueuse, peut être présent au moins partiellement dans la forme tautomère à chaîne overte, ou en un sel de cet acide, et ensuite on hydrogène le composé obtenu; et, dans le cas où on désire un ester ou un sel d'ester, on transforme l'acide libre résultant ou son sel en l'ester ou sel d'ester désiré.

2. Un procédé selon la revendication 1, caractérisé en ce que la matière de départ est un ester d'un acide dont le squellette carbondé comprend l'unité I.

3. Un procédé selon la revendication 2, caractérisé en ce que l'ester est un ester alcoylique ayant 1 à 4 atomes de carbone dans le groupe alcoyle.

4. Un procédé selon l'une des revendications 2 ou 3, caractérisé en ce que l'ester de départ est transformé en un ester de l'acide de formule II soit directement en utilisant un agent oxydant, soit part chloration ou bromation de l'ester de départ de façon à produire en ester d'un acide de formule:

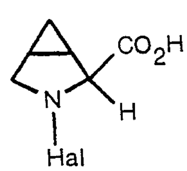

(III)

dans laquelle Hal est un atome de chlore ou de brome, et ensuite déshalogénhydration du composé N-halogéné; et ensuite l'ester résultant est hydrolysé pour produire un acide de formule II ou un sel de cet acide.

5. Un procédé selon la revendication 4, caractérisé en ce que l'ester de départ est chloré en utilisant du N-chloro succinimide, de l'hypochlorite de t-butyle ou de l'hypochlorite de sodium .

6. Un procédé selon la revendication 1, caractérisé en ce qu'un acide libre de formule I ou un sel de cet acide est N-chloré en utilisant de l'hypochlorite de sodium et ensuite déshalogénhydraté pour produire un acide de formule II ou un sel de cet acide.

7. Un procédé selon l'une quelconque des revendications 4 à 6, caractérisé en ce que l'halogénation est conduite à une température comprise entre −10°C et +30°C.

8. Un procédé selon l'une quelconque des revendications 4 à 7, caractérisé en ce qu'on conduit l'étape de déshalogénhydratation en utilisant un hydroxyde ou alcoolate de métal alcalin ou une amine tertiaire.

9. Un prócedé selon l'une quelconque des revendications 4 à 8 caractérisé en ce qu'on conduit l'étape de déshalogénhydratation à une température ne dépassant pas 150°C.

10. Un procédé selon la revendication 4, caractérisé en ce qu'on oxyde directement l'ester de départ en utilisant du bioxyde de manganèse.

11. Un procédé selon l'une quelconque des revendications 1 à 10, caractérisé en ce qu'on conduit l'étape d'hydrogénation en utilisant de l'hydrogène gazeux en présence d'un catalyseur hétérogène.

12. Un procédé selon la revendication 11, caractérisé en ce que la catalyseur comprend du palladium ou du platine.

13. Une composition pour supprimer des pollens que comprend de 2 - carboxy - 3 - azabicyclo[3.1.0]hex - 2 - ène our un sel et/ou un amide, alcoylamide, hydrazide ou alcoylhydrazide de ce composé, en même temps qu'un véhicule.

14. Une composition selon la revendication 12, qui comprend au moins deux véhicules, dont au moins un est un agent tensio-actif.

15. Une méthode pour stériliser les anthéres mâles d'une plante, caractérisé en ce qu'elle comprend l'application à la plante de 2 - carboxy - 3 - azabicyclo[3.1.0]hex - 2 - ène ou d'un sel et/ou d'un amide, alcoylamide, hydrazide out alcoylhydrazide de ce composé, ou d'une composition selon la revendication 13 ou 14.

16. Une méthode pour produire des semences hybrides $F_1$, caractérisé en ce qu'elle comprend la pollinisation croisée d'une plante que a été traitée par un procédé selon la revendication 15 avec une seconde plante d'une souche différente.

17. Un procédé pour la préparation de 2 - carboxy - 3 - azabicyclo[3.1.0]hex - 2 - ène ou d'un sel et/ou d'un amide, alcoylamide, hydrazide ou alcoylhydrazide de ce composé, que comprend l'oxydation du 2 - carboxy - 3 - azabicyclo[3.1.0]hexane ou d'un sel et/ou un ester de ce composé et, si la matière de départ est un ester, l'hydrolyse de l'ester résultant de 2 - carboxy - 3 - azabicyclo[3.1.0]hex - 2 - ène et, dans le cas où on désire un amide, alcoylamide, hydrazide ou alcoylhydrazide, la conversion de l'acide ou du sel résultant in le composé désiré.

18. Un procédé pour la préparation de l'isomère cis du 2 - carboxy - 3 - azabicyclo-[3.1.0]hexane ou d'un sel de ce composé, que comprend l'hydrogénation du 2 - carboxy - 3 - azabicyclo[3.1.0]hex - 2 - ène ou d'un sel de ce composé.

19. Le 2 - carboxy - 3 - azabicyclo[3.1.0]-hex - 2 - ène ou un sel et/ou un amide, alcoylamide, hydrazide ou alcoylhydrazide de ce composé.

20. Le monohydrate de 2 - carboxy - 3 - azabicyclo[3.1.0]hex - 2 - ène.